(19) Europäisches Patentamt
European Patent Office
Office européen des brevets

(11) Publication number: **0 126 881**
**A2**

(12) # EUROPEAN PATENT APPLICATION

(21) Application number: **84103153.7**

(22) Date of filing: **22.03.84**

(51) Int. Cl.³: **A 61 K 7/42**
**C 07 F 9/12**

(30) Priority: **30.03.83 US 480465**

(43) Date of publication of application:
**05.12.84 Bulletin 84/49**

(84) Designated Contracting States:
**AT BE CH DE FR GB IT LI NL SE**

(71) Applicant: **PLOUGH, INC.**
**3030 Jackson Avenue**
**Memphis Tennessee 38151(US)**

(72) Inventor: **Agin, Patricia Poh**
**8556 Kettering Drive**
**Cordova, Tennessee 38018(US)**

(72) Inventor: **Pawelek, John Mason**
**154 Woodlawn Street**
**Hamden, Connecticut 06517(US)**

(74) Representative: **Antony, Fritz, Dr. et al,**
**c/o Scherico Ltd. P.O. Box 601 Töpferstrasse 5**
**CH-6002 Lucerne(CH)**

(54) **Tanning composition.**

(57) The invention relates to compositions useful for increasing melanization in the epidermis of a mammal, comprising a mono- or di-O-phosphorylated derivative of DOPA.

EP 0 126 881 A2

0126881

## TANNING COMPOSITIONS

This invention relates to compositions which increase the natural accumulation of melanins in the skin of humans and other mammals thereby imparting to the skin a natural tan or colouration. Further the invention relates to novel chemical compounds suitable for being included as active ingredients in said compositions as well as to methods for preparing such compositions.

The active ingredients to be used in the compositions of this invention are of the general formula

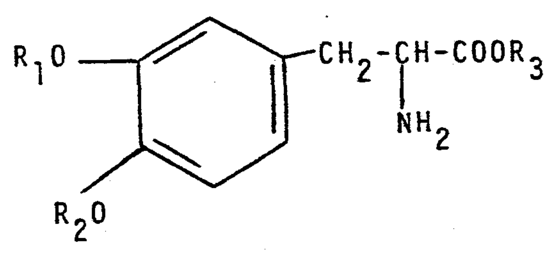

I,

wherein

$R_1$ and $R_2$ independently represent hydrogen or a group $-P(O)-(OR_4)_2$ or $R_1$ and $R_2$ form together the group $>P(O)-(OR_4)$ and

$R_3$ and $R_4$ independently represent hydrogen or a physiologically acceptable cation,

with the proviso that $R_1$ and $R_2$ cannot both be hydrogen.

The compounds of the above formula wherein one of $R_1$ and $R_2$ is hydrogen and the other represents $-P(O)-(OR_4)_2$ or $R_1$ and $R_2$ together form the group $>P(O)-(OR_4)$, $R_3$ and $R_4$ being as above defined, are novel compounds.

Melanins, a class of structurally related chemicals, are the principal pigments of mammalian skin and hair. Melanins are synthesized by specialized cells termed "melanocytes" or "pigment cells", which are found in the skin and hair follicles. Melanocytes respond to ultraviolet radiation, for example, by going through cell divisions resulting in a higher production of melanins. The result of exposure to ultraviolet radiation, e.g. sun-tanning, is therefore an increase in the number of melanocytes and an increase in melanin content of the skin. The ability of human beings to sun-tan is of great medical significance because it has been well-documented that light-skinned individuals are much more susceptible to sun-induced skin cancers than are dark-skinned individuals. Sun-tanning is also of great social significance in that in many parts of the world increased melanin pigmentation is aesthetically desirable. However, for many individuals

sun-tanning is difficult to achieve without concomitant erythema (i.e. sunburn) and resultant peeling of the skin.

Other individuals are unable to achieve an acceptable sun-tan due to their being afflicted with vitiligo which is a disorder of unknown origin which results in the acquired loss of melanocytes in various areas of the skin. The net result of vitiligo is, in the extreme, total depigmentation of skin and hair. More commonly, vitiligo results in patchy, white areas of the skin and hair. In dark-skinned individuals vitiligo can cause severe cosmetic problems and resultant psychological stress. Vitiligo afflicts about 1% or 40 million, of the world's population. A prime treatment for vitiligo is with psoralens and ultraviolet radiation which results in an increase in the number of melanocytes and melanin production in the patchy white areas of the skin. However, psoralens are toxic in that they may cause dermatitis and necrosis when applied to the skin and hepatic insufficiency and nervous and digestive disorders when ingested. There is also evidence that psoralens plus ultraviolet light will induce skin cancer.

Thus, it has long been desired to find a means of effecting an increased accumulation of melanin in the skin and hair of humans and other mammals without the concomitant disadvantages described above.

In order to avoid the aforementioned disadvantages of exposure to the sun, it has been proposed to use solar filters (i.e. sun-screening agents) whereby the amount

of erythema-causing-radiation reaching the skin is diminished. However, in some cases the sun-screening agents reduce the total amount of radiation to the point where the rate of tanning is decreased to an undesirably low level. Thus, many persons desiring a rapid tan are discouraged from using sun screens.

In an attempt to achieve a tanning effect without the above-indicated disadvantages of exposure to UV irradiation and without the use of solar filters, U.S. Patent 2,949,403 proposes using a composition comprising dihydroxyacetone to effect a "simulated" tan on the skin. This composition by effecting the so-called tan through its reaction with the proteinaceous components of the skin obviates the need for exposure to the sun thereby preventing the deleterious effects of such exposure as defined above. However, use of the above composition suffers from numerous disadvantages. It frequently results in different shadings on the skin due to uneven application. Furthermore, the coloured areas are non-uniformly removed during cleaning of the skin.

A number of compositions useful for enhancing colouration of the skin due to exposure to the sun have been proposed, e.g., in U.S. Patent 4,293,542, wherein pyridinyl oxide derivatives are disclosed which function at UV wavelengths outside the erythemal range thus permitting use of sun-screening agents while facilitating rapid tanning without the usual concommitant effects of exposure to the shorter UV wavelengths.

In U.S. Patent 4,228,151, there are disclosed various quinoxaline derivatives which may be used in a similar

manner. However, the above compositions require that melanocytes be present in the epidermis in order that exposure to UV radiation be able to effect the colouration of the epidermis. If, however, such melanocytes are not present in the epidermis, tanning or melanization cannot occur despite administration of the aforementioned composition and exposure to UV radiation. Thus, persons having low, or no, concentrations of melanocytes in the epidermis (e.g. vitiligo victims) could not achieve melanization of the epidermis by using the compositions disclosed in the prior art.

In U.S. Patent 4,021, 538 there are described compositions for "producing pigmentation in .....skin" comprising the salts of aliphatic esters of DOPA or α-methyl DOPA. In order to effect the desired pigmentation the above salts must be oxidized, in alkaline media prior to topical application to the subject or by in vivo oxidation by means of topical application of additional oxidizing agents.

The present invention provides compounds useful in preparing compositions which increase the number of melanocytes and the production of melanin in mammalian skin even in the absence of exposure to UV radiation.

It has been found, in accordance with this invention, that increased natural melanization of the epidermis can be achieved without adverse effects, even in persons having no epidermal melanocytes, by administering to such person a composition comprising a compound as defined by formula I.

The compounds of the compositions of this invention have the further advantage above the DOPA-derivatives referred to above in that they are more stable and, at the same time, more soluble.

The compounds of formula I may be prepared according to processes well-known in the art for preparing similar O-phosphorylated derivatives. The compounds are conveniently prepared by treating DOPA, [3-(3,4-dihydroxyphenyl)-alanin], (preferably L-DOPA) with standard phosphorylating agents.

Typical phosphorylating agents are phosphoroxychloride ($POCl_3$), polyphosphoric acid $(HPO_3)_x$ or, preferably, a mixture of phosphoric acid with phosphorpentoxide ($P_2O_5$). Obviously, when $POCl_3$ is used, the reaction has to be followed by a hydrolysis in accordance with standard procedures. The compounds may be isolated in the form of free acids or as salts.

Typical representatives of physiologically acceptable salts are such formed with metal cations or with organic cations. Representatives of metal cations are sodium, potassium, calcium and magnesium, and of organic groups triethanolamine and tris-(hydroxymethyl)aminomethane. Preferred are the non-oxidizable and non-reducible cations triethanolamine, tris-(hydroxymethyl)-aminomethane, sodium and potassium.

By the phosphorylation reaction described above, a mixture of various phosphorylated compounds is usually obtained, including mono- and di-phosphorylated derivatives of formula I.

The mixture may be used as such in the compositions of this invention, but, if desired, the components may also be separated by means of cation exchange resins. The mono-phosphorylated compounds of formula I and their salts are the most active compounds and, accordingly, are preferred.

The compositions of this invention may be such for oral, topical, transdermal or parenteral application. Thus, the compositions may be in the form of injectable solutions, lotions, creams, ointment, powders etc.

It has been found that the extent of melanization may be intensified if the subject is also exposed to UV irradiation prior to, during or after application of the compositions of this invention. Accordingly a preferred application form is topical preparations suitable for sun-bathing and sun-tanning purposes.

Physiologically acceptable carriers useful in the practice of the invention are known in the art and include, for injection - distilled water; for controlled release - microcapsules comprising carboxymethylene copolymers; for transdermal release - acrylamides and for topical applications - cosmetic bases.

In addition, if desired, the composition according to this embodiment may comprise at least one additive selected from the group consisting of solvents, fragrances, sun-screening agents, preservatives and chelating agents.

Furthermore, as compositions for use in accordance with the embodiment of the invention must be physiologically acceptable it may be desirable to convert the O-phosphorylated DOPA derivatives, if obtained in their free acid form, to their salt forms, e.g., by ion exchange or to add acids, bases or buffers to obtain compounds having the required acidity characteristics.

Cosmetic bases useful in the practice of the invention are well-known and include lotions, creams, ointments, and powders. Examples thereof may be found in e.g., U.S. Patents 4,228,151; 4,282,206 and 2,949,403.

Solvents for use in accordance with the invention include ethanol, distilled and/or deionized water, physiological saline solution and the like. The specific solvent chosen will depend on the method of application.

As the O-phosphorylated DOPA derivatives are good microbiological growth media it is also desirable to add a preservative to compositions comprising those materials if they are to be used for topical applications.

Preservatives are well-known and may be exemplified by methyl-paraben, Dowacil$_{(TM)}$2000 and propylparaben.

Sun-screening agents for topical use in accordance with the invention include most commercially available screening agents especially those described by the

Monograph On Sunscreens, Fed. Register, Part 2, 43 (August 25, 1978) as safe and effective. Additional sun-screening agents are described e.g., in U.S. Patents: 4,264,581; 2,949,403 and 4,256,664.

As compositions comprising the O-phosphorylated DOPA derivatives may be deactivated by reducible or oxidizable cations, such as those of copper or iron, or even by excess amounts of multivalent cations such as calcium or magnesium it is often desirable to have such compositions contain chelating agents, many of which are known in the art, such as ethylenediamine-tetra-acetic acid (EDTA).

If desired, in order to reduce the acidity or basicity of the compositions, bases, acids or buffers may be added thereto in accordance with the knowledge of the art.

The O-phosphorylated DOPA derivatives are usually present in the compositions in concentrations of 0.005 and 1.0% by weight preferably between 0.01 and 0.1% by weight. The daily dose for parenteral or oral application is between 10 mg and 100 mg, preferably between 300 mg and 700 mg per day.

In the following examples parts by weight refer to grams and parts by volume to milliliters.

## EXAMPLE I

### Preparation of O-phosphorylated DOPA derivative

a) To 8.2 ml of concentrated phosphoric acid is added slowly, with stirring 8.4 g of $P_2O_5$. The solution is stirred until a homogenous solution is sustained.

b) To the above mixture, are added 3 g of L-DOPA and the resultant mixture is stirred until the L-DOPA is dissolved.

c) The solution from (b) is heated with stirring at 100°C. for 72 hours, during which the solution becomes darker in colour.

d) The solution from step (c) is then poured over 130 ml of ice and after thawing and mixing, the resulting mixture is poured into a chromatographic column of (6 x 60cm) containing Dowex(TM) 50W-X8 (Biolab, Inc.), of 200-400 mesh, which has been equilibrated with water. The O-phosphorylated DOPA is retained in the column while the excess $H_3PO_4$ and the inorganic salts pass through quickly. After elution of the $H_3PO_4$ and the inorganic salts, the pH of the solution increases. The O-phosphorylated DOPA, which is detected by its UV-absorption at 280 nm, is then eluted.

e) The O-phosphorylated DOPA is then recovered from the pooled eluates by lyophilization and is purified by passage through a chromatographic column containing Dowex (TM) AG7-X8 (Bio-Rad Co) of

200-400 mesh in the chloride form which has been equilibrated with water. It is then eluted with a HCl gradient of about 0 to 150mM . The eluates showing a UV-absorption peak at 280nm , are pooled and lyophilized whereby the desired compound (herein after designated DP) is obtained.

EXAMPLE 2

The procedure of Example 1 is repeated, however, at the elution of the column (step d) the first two fractions are collected which have an absorbence of either 278nm or 254nm depending on the apparatus setting. Since the two compounds will partly elute at the same time, the chromatographic procedure (step e of Example 1) is repeated. After purification two compounds which according to analysis appear to be the 3-mono-O-phosphorylated DOPA and the 4-mono-O-phosphorylated DOPA. The compounds melt at about 220°C. and 180°C., respectively.

## FORMULATIONS

### Formulation I

Injectable composition

A solution is prepared by mixing 500 mg by weight of PD, in the form of its sodium salt, with sufficient sterile water to prepare 2 parts by volume of the final solution. The solution is then placed into 2 ml single dose ampoules.

The above solution is administered to the subject, by injection, once a day at a dose rate of 2 ml per day.

### Formulation II

Controlled Release Oral System

Microcapsules of PD are prepared by spraying PD granules with a solution of carboxymethylene polymers, (such as Carbopol$_{(TM)}$934P) to achieve a coating thickness which will gradually release the PD over an eight hour period.

The above micro-encapsulated granules are combined in hard gelatin capsules to the extent of 500 mg. PD per capsule.

The composition is administered to the subject at a daily dose rate of 500 mg (i.e., one capsule per day) for two to four weeks.

Formulation III

Transdermal Release Composition

An admixture is prepared comprising,

| | pbw |
|---|---|
| Acrylamide copolymer (e.g., Polytrap$_{(TM)}$FLMD 203) | 20 |
| PD | 5 |
| Alcohol | 74.0 |
| Fragrance | 0.1 |

The above mixture is applied to the skin, once a day, preferably in the morning, for two to four weeks.

Formulation IV

Tanning Oil

A. An admixture is prepared by adding in the order indicated:

| | pbw |
|---|---|
| decyloleate | 25.0 |
| isopropyl myristate | 15.0 |
| and propylene glycol dicaprylate/ dicaprate | 5.0 |
| to mineral oil | 54.85 |

B. An admixture is prepared by adding 0.01 pbw PD to 0.01 pbw of Solertan $_{(TM)}$PB-10 (a poly(propylene glycol) lanolin ether.

C. The admixture of part B is added to the admixture of part A and the resultant admixture mixed until homogeneous.

D. The composition of part C is applied to the skin once or twice daily for two to four weeks.

Formulation V

Suntanning Lotion

An admixture containing

| | pbw |
|---|---|
| ICI G-1800 (poly[oxyethylene]21-stearyl ether) | 5.0 |
| isopropyl myristate | 10.0 |
| preservative | 0.1 |
| stearyl alcohol | 2.0 |
| 2-hydroxy-3,3,5,-trimethylhexyl ester of benzoic acid | 8.0 |
| butylated hydroxyanisole | 0.05 |

The above mixture was heated to 70°C and 60 pbw of water, pre-heated to 70°, was added thereto. The resultant mixture was stirred and allowed to cool to room temperature.

To the above mixture was then added a 1% citric acid solution, to achieve a pH of 5.0 after which 0.01 pbw of PD was added as well as sufficient deionized water to yield 100 pbw of lotion.

The above lotion is applied to the skin one-half (1/2) hour prior to exposure to the sun. After swimming, sweating or towelling as well as after each hour of exposure, the lotion is re-applied.

## T E S T

### Melanization of mouse epidermis using the O-phosphorylated DOPA derivative of Example 1

Eight groups of Skh-2 pigmented hairless mice, having no epidermal melanin, (6/group) were painted with a solution of the composition of part A in 0.1M $Tris_{(TM)}$ -25% glycerol buffered at a pH of 7.0. The concentrations of the DOPA derivatives ranged between about 0.005 and about 1.0% wt. Painting, on the dorsal skin of the mice, at a daily rate of $2ml/cm^2$ was effected for 2 weeks. Four of the groups were also subjected to UV irradiaiton, using a filtered PS-20 UV lamp for 10 min/day, three days per week for a total of four weeks. During the irradiation course the painting of the mice skin continued as described above. Liver and brain tissues, as well as skin, were examined histologically. One group of mice was only subjected to irradiaiton as a control. It was found that melanization in the mice treated with the PD alone was greater than in the mice treated only by irradiation. Irradiation, in addition to the treatment with PD, increased the intensity of the melanization.

## CLAIMS

1. Compositions useful for increasing melanization in the epidermis of a mammal comprising as an active ingredient an O-phosphorylated derivative of 3-(3,4-dihydroxy)phenyl-alanine (DOPA) of the general formula I

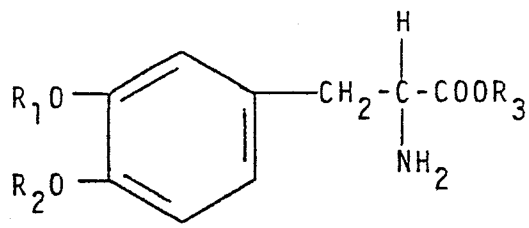

wherein
$R_1$ and $R_2$ represent hydrogen or the group $-P(O)-(OR_4)_2$,
or
$R_1$ and $R_2$ form together the group $\rangle P(O)-(OR_4)_2$,
and
$R_3$ and $R_4$ independently represent hydrogen or a cation, with the proviso that $R_1$ and $R_2$ cannot both be hydrogen.

2. A composition according to claim 1 wherein the O-phosphorylated derivative of formula I is a derivative of L-DOPA.

3. A composition according to either of claims 1 and 2, wherein the active ingredient is a compound of formula I wherein one of $R_1$ and $R_2$ represents hydrogen and the other the group $-P(O)-(OR_4)_2$, $R_4$ being as defined in claim 1.

4. Composition according to any one of claims 1 to 3, wherein the active ingredient is the compound

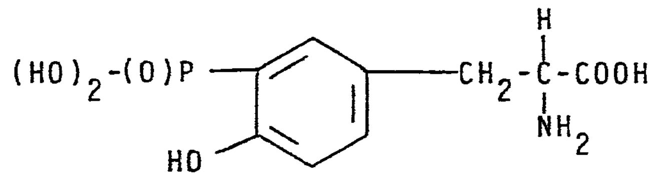

or a salt thereof.

5. Composition according to any one of claims 1 to 3, wherein the active ingredient is a compound of the formula

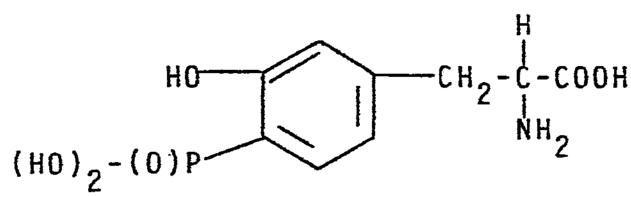

or a salt thereof.

6. A composition according to any one of claims 1 to 5, in a form suitable for topical application useful as a skin tanning composition.

7. A compound of the formula I

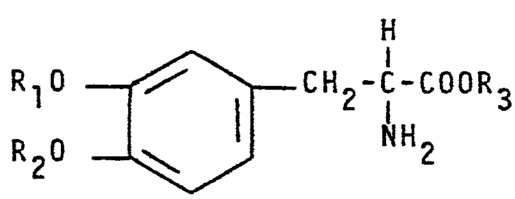

wherein one of $R_1$ and $R_2$ in hydrogen and the other group $-P(O)-(OR_4)_2$ or $R_1$ and $R_2$ represent together the group $P(O)-(OR_4)$, whereby $R_3$ and $R_4$ independently represent hydrogen or a physiologically acceptable cation.

8. The compound of the formula

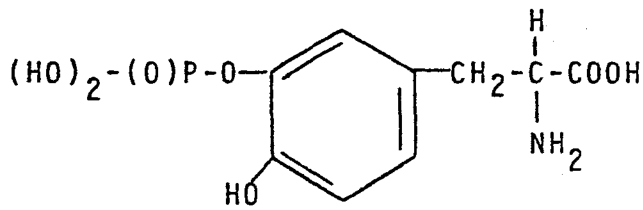

or a salt therof.

9. The compound of the formula

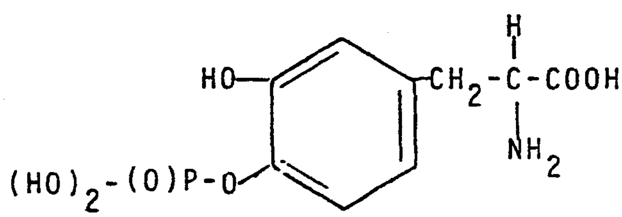

or a salt thereof.

10. Process for preparing a composition as claimed in any one of claims 1 to 6, characterized in that the active ingredients of formula I, or a mixture of such ingredients, is admixed with a suitable carrier.

# CLAIMS

1. Compositions useful for increasing melanization in the epidermis of a mammal, in particular tanning compositions, comprising as an active ingredient an O-phosphorylated derivative of 3-(3,4-dihydroxy)phenylalanine (DOPA) of the general formula I

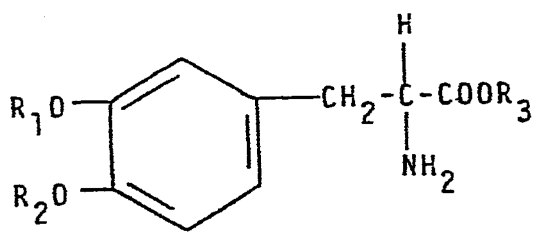

I

wherein

$R_1$ and $R_2$ represent hydrogen or the group $-P(O)-(OR_4)_2$,

or

$R_1$ and $R_2$ form together the group $>P(O)-(OR_4)_2$,

and

$R_3$ and $R_4$ independently represent hydrogen or a cation, with the proviso that $R_1$ and $R_2$ cannot both be hydrogen.

2. A composition according to claim 1 wherein the O-phosphorylated derivative of formula I is a derivative of L-DOPA.

3. A composition according to either of claims 1 and 2, wherein the active ingredient is a compound of formula I wherein one of $R_1$ and $R_2$ represents hydrogen

0126881

Austria

and the other group $-P(O)-(OR_4)_2$, $R_4$ being as defined in claim 1.

4. Composition according to any one of claims 1 to 3, wherein the active ingredient is the compound

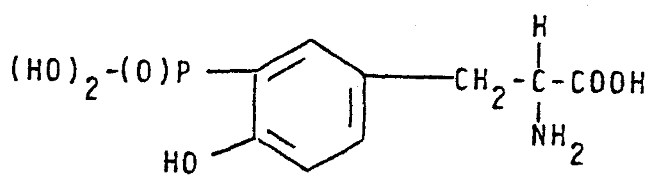

or a salt thereof.

5. Composition according to any one of claims 1 to 3, wherein the active ingredient is a compound of the formula

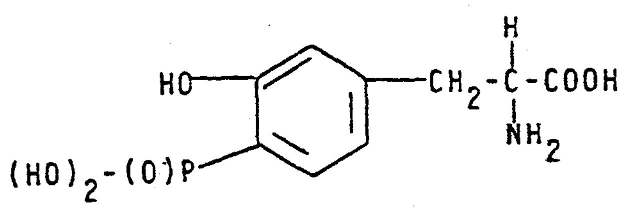

or a salt thereof.

6. A composition according to any one of claims 1 to 5, in a form suitable for topical application useful as a skin tanning composition.

Austria

7. Process for preparing compounds of formula I as set forth and defined in claim 1, said compounds being useful for increasing melanization in the epidermis of a mammal and, in particular, being useful in tanning compositions,
characterized in that a compound of the formula

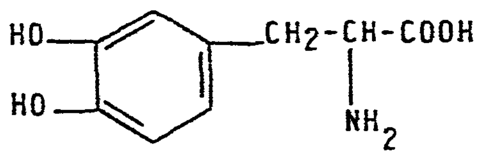

II, (DOPA),

is reacted with a phosphorylating agent and a so-obtained compound is isolated, either in the free form or in the form of a salt.

8. Process for preparing compounds of formula I set forth in claim 1, wherein one of $R_1$ and $R_2$ is hydrogen and the other is a group $-P(O)-(OR_4)_2$, or $R_1$ and $k_2$ form together a group

$$>P(O)—(OR_4)$$

and $R_3$ and $R_4$ independently represent hydrogen or a cation, characterized in that a compound of the formula

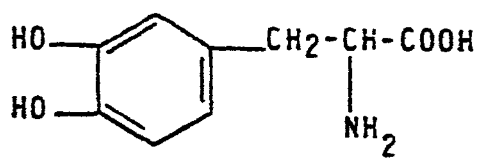

II, (DOPA),

Austria

is reacted with a phosphorylating agent and a so-obtained compound is isolated, either in the free form or in the form of a salt.

9. Process according to claims 7 or 8, characterized in that as a compound of formula II, L-DOPA is used.

10. Process according to claims 7 or 8, characterized in that $POCl_3$ is used as a phosphorylating agent and that the intermediate product is subjected to a hydrolyzation.

11. Process according to claims 7 or 8, characterized in that polyphosphoric acid or a mixture of phosphoric acid and phosphorpentoxide is used as a phosphorylating agent.

12. Process for preparing compositions useful for increasing melanization in the epidermis of a mammal, in particular tanning compositions, characterized in that a compound as defined in any one of claims 1 to 5 is admixed with a suitable carrier.

13. Process for preparing compositions useful for increasing melanization in the epidermis of a mammal, in particular tanning compositions, characterized in that a compound obtained according to any one of claims 7 to 11 is admixed with a suitable carrier.